# EUROPEAN PATENT SPECIFICATION

(11) **EP 2 516 011 B1**
(45) Date of publication and mention of the grant of the patent: **12.07.2017**
(21) Application number: 10838415.7
(22) Date of filing: 22.12.2010
(51) Int. Cl.: A61K 47/16, A61K 47/24, A61K 31/4422, A61K 9/00, A61K 47/10, A61K 47/22, A61K 31/167, A61K 31/196, A61K 31/407, A61K 31/485, A61K 31/573

(54) **CARRIER COMPOSITION FOR DELIVERY OF A BIOLOGICALLY ACTIVE COMPOUND**
ZUBEREITUNG FÜR DEN TRANSPORT VON BIOLOGISCH AKTIVEN SUBSTANZEN
COMPOSITION PORTEUSE POUR LE TRANSPORT D'UN COMPOSÉ ACTIF BIOLOGIQUE

(30) Priority: 23.12.2009 US 289507 P
(43) Date of publication of application: 31.10.2012
(73) Proprietor: Phosphagenics Limited, Clayton, Victoria 3168 (AU)
(72) Inventor: GAVIN, Paul David, Clayton Victoria 3168 (AU); EL-TAMIMY, Mahmoud, Clayton Victoria 3168 (AU); COTTRELL, Jeremy James, Clayton Victoria 3168 (AU); GAETANO, Giacinto, Clayton Victoria 3168 (AU); KENNEDY, Nicholas John, Clayton Victoria 3168 (AU)
(74) Representative: Wakerley, Helen Rachael
(86) International application number: PCT/AU2010/001719
(87) International publication number: WO 2011/075775

(56) References cited:
- WO-A1-02/40033
- WO-A1-03/011303
- WO-A1-03/049774
- WO-A1-2004/014432
- WO-A1-2004/064831
- US-A- 5 387 579
- US-A1- 2009 036 354
- MUNTEANU A ET AL: "Modulation of cell proliferation and gene expression by alpha-tocopheryl phosphates: relevance to atherosclerosis and inflammation", BIOCHEMICAL AND BIOPHYSICAL RESEARCH COMMUNICATIONS, ACADEMIC PRESS INC. ORLANDO, FL, US, vol. 318, no. 1, 21 May 2004 (2004-05-21), pages 311-316, XP004504106, ISSN: 0006-291X, DOI: 10.1016/J.BBRC.2004.04.028
- Paul Gavin: "transdermal delivery", , 14 October 2008 (2008-10-14), pages 34-41, XP055064243, Retrieved from the Internet: URL:http://www.zangani.com/files/2008-1014 -DDT-34-41.pdf [retrieved on 2013-05-27]

## Description

### TECHNICAL FIELD

The present invention relates to carrier compositions for delivery of biologically active compounds.

### BACKGROUND

In this specification where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

Drug delivery is the method or process of administering a pharmaceutical compound to achieve a therapeutic effect in humans and animals.

Drug delivery technologies have been developed to improve bioavailability, safety, duration, onset or release, of the pharmaceutical compound.

When developing drug delivery technologies, problems likely to be encountered include compatibility of the drug delivery system and the pharmaceutical compound, maintaining an adequate and effective duration, potential for side effects, and meeting patient convenience and compliance. As a consequence, many drug delivery technologies fall short of desired improvements and requirements.

Accordingly, there is still a need for alternate drug delivery systems which effectively deliver drugs. US 2009/0036354 discloses carrier compositions for administering biologically active agents comprising C1-C4 alcohols, polyols, and polymers thereof, water and one or more di- and/or mono-(electron transfer agent) phosphate derivatives or complexes thereof.

### SUMMARY

It has surprisingly been found that a carrier composition comprising a phosphate compound of an electron transfer agent and a relatively high concentration of a polar protic solvent can improve the delivery of a biological active compound.

According to a first aspect of the invention there is provided a carrier composition for delivery of a biologically active compound, comprising: i) a phosphate compound of an electron transfer agent which is mixture of a mono-(tocopheryl) phosphate and a di-(tocopheryl) phosphate; and ii) a polar protic solvent, wherein the polar protic solvent is a C₂-C₃ acyclic alcohol; and the polar protic solvent concentration is within the range of 60%w/w to 90%w/w of the total concentration of the carrier composition; wherein the biologically active compound is lipophilic having a log*P* value within the range of 1 to 5, has a molecular mass of less than 1000 Daltons and has a melting point of less than 400 °C.

There is also disclosed use of a phosphate compound of an electron transfer agent and a polar protic solvent in the manufacture of the carrier composition.

There is further disclosed a process for the preparation of the carrier composition which comprises the step of combining a phosphate compound of an electron transfer agent and a polar protic solvent until complete homogenisation is achieved.

The polar protic solvent concentration is within the range of preferably about 65%w/w to about 85%w/w, most preferably about 70%w/w to about 80%w/w. In some circumstances, a suitable range may be about 60%w/w to about 70%w/w or about 80%w/w to about 90%w/w.

Also disclosed herein is a polar protic solvent, which is an acyclic alcohol selected from the group consisting of C₁-C₄ acyclic alcohols, polyols of C₁-C₄ acyclic alcohols, polymers of C₁-C₄ acyclic alcohols, and ester, alkyl ester and ether derivatives thereof. More particularly, an acyclic alcohol may be ethanol, n-propanol, isopropanol, ethylene glycol, propylene glycol, polyethylene glycol, diethylene glycol monoethylether or ethyl acetate.

A polar protic solvent may be a ketone. More particularly, the ketone may be methyl isobutyl ketone or acetone.

A polar protic solvent may be a nitrile. More particularly, the nitrile may be acetonitrile.

An electron transfer agent may be an antioxidant or a derivatised compound thereof. An electron transfer agent may be a hydroxy chroman, e.g. a tocol such as tocopherol or tocotrienol.

Phosphate compounds of tocopherol may be selected from the group consisting of mono-(tocopheryl) phosphate, mono-(tocopheryl) phosphate monosodium salt, mono-(tocopheryl) phosphate monopotassium salt, mono-(tocopheryl) phosphate disodium salt, mono-(tocopheryl) phosphate dipotassium salt, di-(tocopheryl) phosphate, di-(tocopheryl) phosphate monosodium salt, di-(tocopheryl) phosphate monopotassium salt, or a mixture thereof.

The carrier composition of the invention comprises a mixture of a mono-(tocopheryl) phosphate and a di-(tocopheryl) phosphate. Optionally the ratio is at least 2:1, more preferably within the range of about 4:1 to about 1:4, most preferably within the range of about 6:4 to about 8:2. In preferred embodiments the ratio is about 6:4 or about 8:2.

The carrier composition comprises a phosphate compound of an electron transfer agent in an amount within the range of preferably about 0.01%w/w to about 20%w/w, more preferably about 0.01%w/w to about 10%w/w, most preferably about 0.01%w/w to about 5%w/w or about 0.01 %w/w to about 1%w/w of the total concentration of the carrier composition. In one embodiment the carrier composition comprises a phosphate compound of an electron transfer agent in an amount within the range of about 0.01%w/w to about 2%w/w, preferably about 0.05%w/w, about 0.1%w/w or about 1%w/w. In a further embodiment, a range of about 5%w/w to about 10%w/w or about 10%w/w to about 15%w/w may be used.

In a second aspect of the invention there is provided a formulation comprising the carrier composition and a biologically active compound, wherein the biologically active compound is lipophilic having a log*P* value within the range of 1 to 5 and has a molecular mass of less than about 1000 Daltons and/or a melting point of less than about 400 °C.

There is also disclosed a process for the preparation of the formulation which comprises the step of adding a biologically active compound to the carrier composition. In one embodiment, the biologically active compound is lipophilic having a log*P* value within the range of about 1 to about 5. The biologically active compound also preferably has a relatively low molecular mass and a relatively low melting point.

A biologically active compound may be present in an amount of up to about 30%w/w of the total concentration of the carrier composition.

In a third aspect of the invention there is provided use of the carrier composition to improve the delivery of a biologically active compound formulated with the carrier composition.

There is also provided use of the carrier composition to alter A.D.M.E. properties of a biologically active compound.

There is further provided use of the carrier composition to improve the bioavailability of a biologically active compound in a subject.

Also disclosed herein is a method for treating a subject for a pathological condition which comprises administering an effective amount of a biologically active compound in the carrier composition. The pathological conditions include those that can be treated by the biologically active compound formulated with the carrier composition.

### DETAILED DESCRIPTION

A carrier composition of the present invention comprises a phosphate compound of an electron transfer agent and a relatively high concentration of a polar protic solvent. A biologically active compound may be formulated with a carrier composition of the present invention to provide a formulation.

In this specification, except where the context requires otherwise, the words "comprise", "comprises", and "comprising" mean "include", "includes", and "including" respectively, i.e. when the invention is described or defined as comprising specified features, various embodiments of the same invention may also include additional features.

### Phosphate compound of an electron transfer agent

The term "electron transfer agent" refers to a compound that may be phosphorylated and which, in the non-phosphorylated form, can accept an electron to generate a relatively stable molecular radical or can accept two electrons to allow the compound to participate in a reversible redox system. Examples of electron transfer agents include antioxidants and derivatives thereof.

The term "antioxidant" refers to a molecule capable of slowing or preventing the oxidation of other molecules. Oxidation is a chemical reaction that transfers electrons from a substance to an oxidizing agent. Oxidation reactions can produce free radicals, which start chain reactions that damage cells. Antioxidants terminate these chain reactions by removing free radical intermediates, and inhibit other oxidation reactions by being oxidized themselves. As a result, antioxidants are often reducing agents.

Antioxidants are generally classified into two broad divisions, depending on whether they are soluble in water (hydrophilic) or in lipids (hydrophobic). Ascorbic acid (vitamin C) is an example of a water soluble antioxidant. Carotenes, tocopherol (Vitamin E), retinol (Vitamin A), ubiquinol (the reduced form of coenzyme Q) and calciferol (Vitamin D) are examples of lipid soluble antioxidants.

Carotenes are carotenoids containing no oxygen. Carotenoids are based on carotenes with one or more hydrogen atoms substituted by a hydroxyl group and/or some pairs of hydrogen atoms are substituted by oxygen atoms. The term "hydroxy carotenoids" refers to carotenes substituted with one or more hydroxyl groups. Cryptoxanthin is an example of a hydroxy carotenoid: it is closely related to beta-carotene with only the addition of a hydroxyl group.

Vitamin E exists in eight different forms, namely four tocopherols and four tocotrienols. All feature a chroman ring, with a hydroxyl group that can donate a hydrogen atom to reduce free radicals and a hydrophobic side chain which allows for penetration into biological membranes. Such derivatives of Vitamin E may be classified as "hydroxy chromans". Both tocopherols and tocotrienols occur in alpha, beta, gamma and delta forms, determined by the number and location of methyl groups on the chroman ring. The tocotrienols differ from the analogous tocopherols by the presence of three double bonds in the hydrophobic side chain. The various forms of Vitamin E are shown by Formula (I):

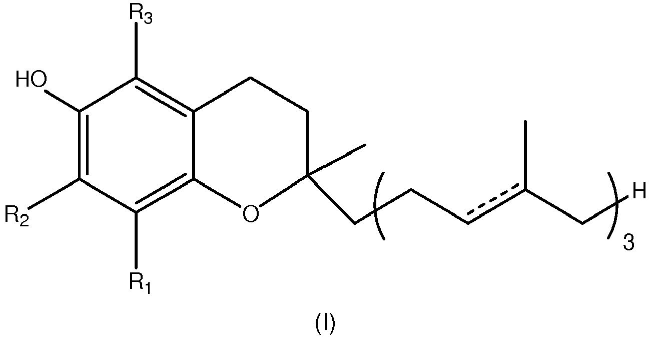

| | R₁ | R₂ | R₃ |
|---|---|---|---|
| α-tocopherol | CH₃ | CH₃ | CH₃ |
| α-tocotrienol | CH₃ | CH₃ | CH₃ |
| β-tocopherol | CH₃ | H | CH₃ |
| β-tocotrienol | CH₃ | H | CH₃ |
| γ-tocopherol | H | CH₃ | CH₃ |
| γ-tocotrienol | H | CH₃ | CH₃ |
| δ-tocopherol | H | H | CH₃ |
| δ-tocotrienol | H | H | CH₃ |

Retinol belongs to the family of chemical compounds known as retinoids. There are three generations of retinoids. First generation retinoids include retinol, retinal, tretinoin (retinoic acid, Retin-A), isotretinoin and alitretinoin. Second generation retinoids include etretinate and its metabolite acitretin. Third generation retinoids include tazarotene, bexarotene and adapalene.

Ubiquinol is a benzoquinol and is the reduced form of ubiquinone (coenzyme Q₁₀).

Calciferol (Vitamin D) comes in several forms. The two major forms are vitamin D₂ (e.g. ergocalciferol) and vitamin D₃ (e.g. calcitriol, cholecalciferol). The other forms include vitamin D₁ (molecular compound of ergocalciferol with lumisterol, 1:1), vitamin D₄ (22-dihydroergocalciferol) and vitamin D5 (sitocalciferol, made from 7-dehydrositosterol).

Any antioxidant or derivative thereof described herein would be suitable for the present disclosure. Preferred antioxidants and derivatives thereof are selected from the group consisting of carotenoids, hydroxy chromans, carotenoids, retinoids, benzoquinols and calcitriols. Hydroxy chromans are preferred. Tocols such as a tocopherol, in any form, is most preferred.

The term "phosphate compound" refers to a phosphorylated compound, where a covalent bond is formed between an oxygen atom (typically originating from a hydroxyl group) of the compound and the phosphorous atom of a phosphate group (PO₄): in this context, the compound is an electron transfer agent.

A phosphate compound may be a phosphate mono-ester, phosphate di-ester, phosphate tri-ester, pyrophosphate mono-ester, pyrophosphate di-ester, or a salt or derivative thereof, or a mixture thereof. The di- and tri-esters may comprise the same electron transfer agent or different electron transfer agents.

The "salts" include metal salts such as alkali or alkaline earth metal salts, for example sodium, magnesium, potassium and calcium salts. Sodium and potassium salts are preferred.

The "derivatives" include phosphate compounds where one or more phosphate protons are replaced by a substituent. Some non-limiting examples of derivatives include phosphatidyl derivatives where a phosphate proton is substituted with an amino-alkyl group, sugar derivatives where a phosphate proton is substituted with a sugar such as glucose.

The term "amino-alkyl group" refers to a group comprising an amino (-NH₂) group and an alkyl group. The term "alkyl" refers to straight chain, branched chain or cyclic hydrocarbon groups having from 1 to 8 carbon atoms. Examples include methyl, ethyl, propyl, isopropyl, butyl, isobutyl, sec-butyl, tert-butyl, pentyl, hexyl, cyclohexyl, heptyl, and octyl. Phosphatidyl choline derivatives are most preferred.

When the electron transfer agent is tocopherol, for example, the phosphate compounds of tocopherol may be selected from the group consisting of mono-(tocopheryl) phosphate, mono-(tocopheryl) phosphate monosodium salt, mono-(tocopheryl) phosphate monopotassium salt, mono-(tocopheryl) phosphate disodium salt, mono-(tocopheryl) phosphate dipotassium salt, di-(tocopheryl) phosphate, di-(tocopheryl) phosphate monosodium salt, di-(tocopheryl) phosphate monopotassium salt, or a mixture thereof. These phosphate compounds may be derived from the alpha, beta, gamma or delta form of tocopherol, or a combination thereof.

The carrier composition contains a mixture of a mono-(tocopheryl) phosphate and di-(tocopheryl) phosphate, and the ratio may be at least 2:1, more preferably within the range of about 4:1 to about 1:4, most preferably within the range of about 6:4 to about 8:2. The ratio may be about 6:4 or about 8:2.

The carrier composition comprises a phosphate compound of an electron transfer agent in an amount within the range of preferably about 0.01%w/w to about 20%w/w, more preferably about 0.01%w/w to about 10%w/w, most preferably about 0.01%w/w to about 5%w/w or about 0.01 %w/w to about 1%w/w of the total concentration of the carrier composition. The carrier composition may comprise a phosphate compound of an electron transfer agent in an amount within the range of about 0.01%w/w to about 2%w/w, preferably about 0.05%w/w, about 0.1%w/w or about 1%w/w. In a further embodiment, a range of about 5%w/w to about 10%w/w or about 10%w/w to about 15%w/w may be suitable.

### Polar protic solvent

Organic solvents may be grouped as non-polar or polar aprotic solvents, and polar protic solvents.

The carrier composition of the present invention comprises a polar protic solvent. A "protic solvent" is a solvent that has a hydrogen atom bound to an oxygen atom or a nitrogen atom as in a hydroxyl group or an amine group, respectively. More generally, any molecular solvent which contains a hydrogen ion capable of dissociation may be considered a protic solvent. Conversely, an "aprotic solvent" cannot donate hydrogen ions.

A polar protic solvent of the disclosure may be an acyclic alcohol, alkyl ester, ketone or nitrile. An acyclic alcohol may be selected from the group consisting of C₁-C₄ acyclic alcohols including polyols, polymers and derivatives (e.g. esters, alkyl esters, ethers) thereof. Some examples of these include ethanol, n-propanol, isopropanol, glycols such as propylene glycol, polyethylene glycol (e.g. PEG400), diethylene glycol monoethylether and ethyl acetate. A ketone may be selected from the group consisting of methyl isobutyl ketone and acetone. A nitrile may be acetonitrile.

The carrier composition may comprise only one polar protic solvent; however, a combination of polar protic solvents may also be used. For the avoidance of any doubt, it is to be noted that the singular forms "a", "an" and "the" as used herein for any feature should be read as encompassing plural forms, unless the context clearly indicates otherwise.

The carrier composition has a relatively high polar protic solvent concentration. The polar protic solvent concentration is within the range of preferably about 60%w/w to about 90%w/w, more preferably about 65%w/w to about 85%w/w, most preferably about 70%w/w to about 80%w/w. The polar protic solvent concentration may be about 70%w/w or about 80%w/w. In some circumstances a suitable range of polar protic solvent concentration may be about 60%w/w to about 70%w/w or about 80%w/w to about 90%w/w.

### Biologically active compound

The term "biologically active compound" refers to any chemical substance that has a biological effect in humans or animals for medical, therapeutic, cosmetic and veterinary purposes, and encompasses pharmaceuticals including drugs, cosmeceuticals, nutraceuticals, and nutritional agents. It will be appreciated that some of biologically active compounds can be classified in more than one of these classes.

A wide range of biologically active compounds may be delivered with a carrier composition of the present invention. The biologically active compound is lipophilic having a log*P* value within the range of 1 to 5, and has a relatively low molecular mass of less than about 1000 Daltons and a relatively low melting point of less than about 400°C.

The term "lipophilicity" refers to the ability of a chemical compound to dissolve in fats, oils, lipids, and non-polar solvents such as hexane or toluene. The lipophilicity of a biologically active compound may be assessed by its octanol/water partitioning coefficient (Iog*P* value), which is believed to approximate membrane permeability.

Transdermally permeable biologically active compounds are likely to have a log*P* value between about 2.5 to about 3.5. It has been found that biologically active compounds having a log*P* value within this range and biologically active compounds having a log*P* value outside this range (either above or below) formulated with a carrier composition of the present invention are more transdermally permeable, that is, biologically active compounds having a log*P* value within the range of about 1 to about 5. Therefore, the biologically active compound may have a log*P* value within the range of about 1 to about 2.5, within the range of about 2.5 and about 3.5, and within the range of about 3.5 to about 5.

Not wishing to be bound by theory, it is believed that a carrier composition of the present invention can alter one or more A.D.M.E. (Absorption, Distribution, Metabolism, and Excretion) properties of a biological active compound to improve the delivery of the biological active compound. To be an effective biological active compound, the biological active compound not only must be active against a target, but also possess the appropriate A.D.M.E. properties necessary to make it suitable for use as a biological active compound.

A "relatively low molecular mass" refers to a molecular mass of less than about 1000 Daltons, preferably less than about 500 Daltons, more preferably within the range of about 200 Daltons to about 300 Daltons.

A "relatively low melting point" refers to a melting point of less than about 400°C.

A wide range of biologically active compounds may be delivered with the carrier composition of the present invention. Examples include, but are not limited to, cardiovascular drugs, in particular antihypertensive agents (e.g. calcium channel blockers (or calcium antagonists)) and antiarrhythmic agents; congestive heart-failure pharmaceuticals; inotropic agents; vasodilators; ACE inhibitors; diuretics; carbonic anhydrase inhibitors; cardiac glycosides; phosphodiesterase inhibitors; α blockers; β blockers; sodium channel blockers; potassium channel blockers; β-adrenergic agonists; platelet inhibitors; angiotensin II antagonists; anticoagulants; thrombolytic agents; treatments for bleeding; treatments for anaemia; thrombin inhibitors; antiparasitic agents; antibacterial agents; antiinflammatory agents, in particular non-steroidal antiinflammatory agents (NSAIDs), more particularly COX-2 inhibitors; steroidal antiinflammatory agents; prophylactic antiinflammatory agents; antiglaucoma agents; mast cell stabilisers; mydriatics; agents affecting the respiratory system; allergic rhinitis pharmaceuticals; alpha-adrenergic agonists; corticosteroids; chronic obstructive pulmonary disease pharmaceuticals; xanthine-oxidase inhibitors; antiarthritis agents; gout treatments; autacoids and autacoid antagonists; antimycobacterial agents; antifungal agents; antiprotozoal agents; anthelmintic agents; antiviral agents especially for respiratory , herpes, cyto-megalovirus, human immunodeficiency virus and hepatitis infections; treatments for leukemia and kaposi's sarcoma; pain management agents in particular anaesthetics and analgesics, opioids including opioid receptor agonists, opioid receptor partial agonists, opioid antagonist or opioid receptor mixed agonist-antagonists; neuroleptics; sympathomimetic pharmaceuticals; adrenergic agonists; drugs affecting neurotransmitter uptake or release; anticholinergic pharmaceuticals; antihaemorrhoid treatments; agents to prevent or treat radiation or chemotherapeutic effects; liopgenisis drugs; fat reducing treatments; antiobesity agents such as lipase inhibitors; sympathomimetic agents; treatments for gastric ulcers and inflammation such as proton pump inhibitors; prostaglandins; VEGF inhibitors; antihyperlipidemic agents, in particular statins; drugs that affect the central nervous system (CNS) such as antipsychotic, antiepileptic and antiseizure drugs (anticonvulsants), psychoactive drugs, stimulants, antianxiety and hypnotic drugs, antidepressant drugs; antiparkinson's pharmaceuticals; hormones and fragments thereof such as sex hormones; growth hormone antagonists; gonadotropin releasing hormones and analogues thereof; steroid hormones and their antagonists; selective estrogen modulators; growth factors; antidiabetic pharmaceuticals such as hypoglycaemic agents; H1, H2, H3 and H4 antihistamines; agents used to treat migraine headaches; asthma pharmaceuticals; cholinergic antagonists; glucocorticoids; androgens; antiandrogens; inhibitors of adrenocorticoid biosynthesis; osteoporosis treatments such as biphosphonates; antithyroid pharmaceuticals; suncreens, sun protectants and filters; cytokine agonists; cytokine antagonists; anticancer drugs; antialzheimer drugs; HMGCoA reductase inhibitors; fibrates; cholesterol absorption inhibitors; HDL cholesterol elevating agents; triglyceride reducing agents; antiageing or antiwrinkle agents; antibacterial agents; antiacne agents; antioxidants; hair treatments and skin whitening agents; small molecule therapeutic agents for the treatment, or prevention of human and animal diseases such as allergy/asthma, arthritis, cancer, diabetes, growth impairment, cardiovascular diseases, inflammation, immunological disorders, baldness, pain, ophthalmological diseases, epilepsy, gynaecological disorders, CNS diseases, viral infections, bacterial infections, parasitic infections, GI diseases, obesity, and haemological diseases.

A person skilled in the art of the invention would be able to determine whether or not a particular biologically active compound would be suitable for use with the carrier composition of the present invention. Some specific non-limiting examples of suitable biologically active compounds include:
***anaesthetics:***
   including amino-ester and amino-amide anaesthetics such as benzocaine, chloroprocaine, cocaine, reserpine, guanethidine, cyclomethycaine, dimethocaine/larocaine, propoxycaine, procaine/novocaine, proparacaine, tetracaine/amethocaine; articaine, bupivacaine, carticaine, cinchocaine/dibucaine, etidocaine, levobupivacaine, lidocaine/lignocaine, mepivacaine, piperocaine, prilocaine, ropivacaine, trimecaine, propofol, halothane, enflurane barbiturates, benzodiazepines, neostigmine and ketamine
***alkylating agents:***
   including carmustine, cyclophosphamide, ifosfamide, streptozotocin and mechlorethamine
***calcium channel blockers:***
   including amlodipine, aranidipine, azelnidipine, barnidipine, benidipine, cilnidipine, clevidipine, cronidipine, darodipine, dexniguldipine, efonidipine, elnadipine, elgodipine, felodipine, flordipine, furnidipine, iganidipine, isradipine, lacidipine, lemildipine, lercanidipine, manidipine, mesuldipine, nicardipine, nifedipine, niludipine, nilvadipine, nimodipine, nisoldipine, nitrendipine, olradipine, oxodipine, palonidipine, pranidipine, sagandipine, sornidipine, teludipine, tiamdipine, trombodipine, watanidipine, verapamil, gallopamil, benzothiazepine, diltiazem, mibefradil, bepridil, fluspirilene and fendiline
***antiarrhythmic and antiangina agents**:*
   including amiodarone, disopyramide, flecainide acetate, quinidine sulphate, nitroglycerine, ranolazine, amiodarone, isosorbide and alteplase
***antibacterial, antibiotic and antiacne agents:***
   including amoxicillin, ampicillin, azithromycin, benethamine penicillin, bleomycin, benzoyl peroxide, cinoxacin, chloramphenicol, daunorubicin, plicamycin, fluoroquinolones, ciprofloxacin, clarithromycin, clindamycin, clindesse, clofazimine, chlorohexidine gluconate, cloxacillin, demeclocycline, doxycycline, erythromycin, ethionamide, imipenem, indomethacin, lymocycline, minocycline, nalidixic acid, nitrofurantoin, penicillin, rifampicin, spiramycin, sodium sulfacetamide, sulphabenzamide, sulphadoxine, sulphamerazine, sulphacetamide, sulphadiazine, sulphafurazole, sulphamethoxazole, sulphapyridine, tetracycline, cephalexin, cefdinir, triclosan, ofloxacin, vancocin, glyburide, mupirocin, cefprozil, cefuroxime axetil, norfloxacin, isoniazid, lupulone, D-penicillamine, levofloxacin, gatifoxacin, and trimethoprim
***anticancer:***
   including doxorubicin, 6-thioguanine, paclitaxel, docetaxel, camptothecin, megestrol acetate, navelbine, cytarabine, fludarabine, 6-mercaptopurine, 5-fluorouracil, teniposide, vinblastine, vincristine, cisplatin, colchicine, carboplatin, procarbazine and etopside
***antidepressants, antipsychotics and antianxiety:***
   including alprazolam, amoxapine, bentazepam, bromazepam, clorazipine, clobazam, clotiazepam, diazepam, lorazepam, flunitrazepam, flurazepam, lormetazepam, medazepam, nitrazepam, oxazepam, temazepam, maprotiline, mianserin, nortriptyline, risperidone, sertraline, trazodone, baloperidol, trimipramine maleate fluoxetine, ondansetron, midazolam, chlorpromazine, haloperidol, triazolam, clozapine, fluopromazine, fluphenazine decanoate, fluanisone, perphenazine, pimozide, prochlorperazine, sulpiride, thioridazine, paroxitine, citalopram, bupropion, phenelzine, olanzapine, divalproex sodium and venlafaxine
***opioids:***
   including opioid receptor agonists and antagonists, compounds which exhibit mixed agonist/antagonist activity and compounds which exhibit partial agonist activity, including morphine, depomorphine, etorphine, diacetylmorphine, hydromorphone, oxymorphone, levorphanol, methadone, levomethadyl, meperidine, fentanyl, sufentanyl, alfentanil, codeine, hydrocodone, oxycodone, thebaine, desomorphine, nicomorphine, dipropanoylmorphine, benzylmorphine, ethylmorphine, pethidine, methadone, tramadol, dextropropoxyphene; naloxone and naltrexone; buprenorphine, nalbuphine, butorphanol, pentazocine and ethylketocyclazocine
***tricyclics:***
   including azothiopine, amitriptyline, famotidine, promethazine, paroxatine, oxcarbazapine and mertazapine
***antidiabetics:***
   including acetohexamide, chlorpropamide, glibenclaraide, gliclazide, glipizide, metformin, tolazamide, glyburide, glimepiride and tolbutamide
***antiepileptics:***
   including beclamide, carbamazepine, gapapentin, tiagabine, vigabatrin, topiramate, clonazepam, ethotoin, methoin, methsuximide, methylphenobarbitone, oxcarbazepine, paramethadione, phenacemide, phenobarbitone, phenyloin, phensuximide, primidone, sulthiamine, phenytoin sodium, nirofurantoin monohydrate, gabapentin, lamotrigine, zonisamide, ethosuximide and valproic acid
***hypnotics*/*sedatives and muscle relaxants:***
   including zolpidem tartrate, amylobarbitone, barbitone, butobarbitone, pentobarbitone, brotizolam, carbromal, chlordiazepoxide, chlormethiazole, ethinamate, meprobamate, methaqualome, cyclobenzaprene, cyclobenzaprine, tizanidine, baclofen, butalbital, zopiclone, atracurium, tubocurarine and phenobarbital
***antifungal, antiprotazoal and antiparasitic agents**:*
   including amphotericin, butoconazole nitrate, clotrimazole, econazole nitrate, fluconazole, flucytosine, griseofulvin, itraconazole, ketoconazole, miconazole, natamycin, nystatin, sulconazole nitrate, terconazole, tioconazole and undecenoic acid; benznidazole, clioquinol, decoquinate, diiodohydroxyquinoline, diloxanide furoate, dinitolmide, furzolidone, metronidazole, nimorazole, nitrofurazone, ornidazole, terbinafine, clotrimazole, chloroquine, mefloquine, itraconazole, Pyrimethamine, praziquantel, quinacrine, mebendazole and tinidazole
***antihypertensive and cardiac therapeutic agents**:*
   including candesartan, hydralazine, clonidine, triamterene, felodipine, gemfibrozil, fenofibrate, nifedical, prazosin, mecamylamine, doxazosin, dobutamine and cilexetil
***antimigraine agents**:*
   including dihydroergotamine mesylate, ergotamine tartrate, methysergide maleate, pizotifen maleate and sumatriptan succinate
***antimuscarinic agents**:*
   including atropine, benzhexol, biperiden, ethopropazine, hyoscyamine, mepenzolate bromide, oxybutynin, oxyphencylcimine and tropicamide
***antineoplastic agents (or immunosuppressants):***
   including aminoglutethimide, amsacrine, azathioprine, busulphan, chlorambucil, cyclosporin, dacarbazine, estramustine, etoposide, lomustine, melphalan, mercaptopurine, methotrexate, mitomycin, mitotane, mitozantrone, procarbazine, tamoxifen citrate, testolactone, tacrolimus, mercaptopurine and sirolimus
***antiparkinsonian agents**:*
   including bromocriptine mesylate, levodopa, tolcapone, ropinirole, bromocriptine, hypoglycaemic agents such as sulfonylureas, biguanides, α-glucosidase inhibitors, thaiazolidinediones, cabergoline, carbidopa and lysuride maleate
***antithyroid agents**:*
   including carbimazole and propylthiouracil
***antiviral drugs:***
   including amantadine, retinovir, cidofovir, acyclovir, famciclovir, ribavirin, amprenavir, indinavirm, rimantadine and efavirenz, penciclovir, ganciclovir, vidarabine, abacavir, adefovir, apmrenavir, delavirdine, didanosine, stavudine, zalcitabine, zidovudine, enfuvirtide and interferon
***cardiac inotropic agents**:*
   including amrinone, milrinone, digitoxin, digoxin, enoximone, lanatoside C and medigoxin
***hypo and hyper lipidemic agents**:*
   including fenofibrate, clofibrate, probucol, ezetimibe and torcetrapib
***antiinflammatory:***
   including meoxicam, triamcinolone, cromolyn, nedocromil, hydroxychloroquine, montelukast, zileuton, zafirlukast and meloxicam
***antihistamine:***
   including fexofenadine, chloral hydrate, hydroxyzine, promethazine, cetirazine, cimetidine, clyclizine, meclizine, dimenhydrinate, loratadine, nizatadine and promethazine
***antiulcer:***
   including omeprazole, lansoprazole, pantoprazole and ranitidine
*diuretics:*
   including hydrochlorothiazide, amiloride, acetazolamide, furosemide and torsemide
***NSAIDs:***
   including arylalkanoic acid sub-group of class which includes diclofenac, aceclofenac, acemetacin, alclofenac, bromfenac, etodolac, indometacin, indometacin farnesil, nabumetone, oxametacin, proglumetacin, sulindac and tolmetin; 2-arylpropionic acid (profens) sub-group of class which includes alminoprofen, benoxaprofen, carprofen, dexibuprofen, dexketoprofen, fenbufen, fenoprofen, flunoxaprofen, flurbiprofen, ibuprofen, ibuproxam, indoprofen, ketoprofen, ketorolac, loxoprofen, miroprofen, naproxen, oxaprozin, pirprofen, suprofen, tarenflurbil and tiaprofenic acid; and *N*-arylanthranilic acid (fenamic acid) sub-group of class which includes flufenamic acid, meclofenamic acid, mefenamic acid and tolfenamic acid; tromethamine, celecoxib, nepafenac, aspirin, rofecoxib, naproxen, sulindac, piroxicam, pheylbutazone, tolmetin, indomethacin, acetominophen (paracetamol), tramadol and propoxyphene
***retinoids:***
   including first generation retinoids such as retinol, retinal, tretinoin (retinoic acid, Retin-A), isotretinoin and alitretinoin; second generation retinoids such as etretinate and its metabolite acitretin; third generation retinoids such as tazarotene, bexarotene and adapalene
***hormones and steroids:***
   including adrenocorticotrophic hormone (ACTH), antidiruetic hormone (vasopressin), atrial-nartreuretic factor (ANF), beclomethasone, cortisone, scopolamine, dopamine, epinephrine, catecholamines, cholecystokinin, clomiphene citrate, danazol, dexamethasone, diethylstilbestrol (DES), ethinyl estradiol, fludrocortison, finasteride, follicle stimulating hormone, gastrin, hydroxyprogesterone, leptin, luteinizing hormone, medroxyprogesterone acetate, mestranol, quinestrol, methyltestosterone, nandrolone, norethindrone, norethisterone, norgestrel, estradiol, conjugated oestrogens, oxandrolone, oxytocin, prednisone, progesterone, prolactin, protogalndins, somatostatin, stanozolol, stibestrol, thyroxine, prednisolone phosphate, triamcinolone, mifepristone acetonide, budesonide, levothyroxine, testosterone, testosterone cypionate, fluoxymesterone, flutamide, mometasone furoate, cyproterone, fluromethalone, goserelin, leuprolide, calcitonin, halobetasol, hydrocortisol and tibolone
***statins and derivatives:***
   including atorvastatin, fluvastatin, lovastatin, nystatin, rosuvastatin, pravastatin, orlistat and simvastatin
***stimulants:***
   including amphetamine, phentermine, tyramine, ephedrine, metaraminol, phenylephrine, dexamphetamine, dexfenfluramine, fenfluramine, nicotine, caffeine and mazindol
***vasocontrictors:***
   including desmopressin
***vasodilitors:***
   including carvedilol, terazosin, phentolamine and menthol
***antialzheimers:***
   including levetiracetam, levitiracetam and donepezil
***ACE inhibitors:***
   including benzapril, enalapril, ramipril, fosinopril sodium, lisinopril, minoxidil, isosorbide, rampril and quinapril
***beta adrenoreceptor antogonists:***
   including atenolol, timolol, pindolol, propanolol hydrochloride, bisoprolol, esmolol, metoprolol succinate, metoprolol and metoprolol tartrate
***angiotensin II antagonists:***
   including losartan
***platelet inhibitors:***
   including abciximab, clopidrogel, tirofiban and aspirin
***alcohols and phenols:***
   including tramadol, tramadol hydrochloride, allopurinol, calcitriol, cilostazol, soltalol, urasodiol bromperidol, droperidol, flupenthixol decanoate, albuterol, albuterol sulphate, carisoprodol, chlobetasol, ropinirol, labetalol, and methocarbamol
***ketones and esters:***
   including amioderone, fluticasone, spironolactone, prednisone, triazodone, desoximetasone, methyl prednisdone, benzonatate nabumetone and buspirone
***antiemetics:***
   including metoclopramide
***ocular treatments:***
   including dorzolamide, brimonidine, olopatadine, cyclopentolate, pilocarpine and echothiophate
***anticoagulant and antithrombitic agents:***
   including warfarin, enoxaparin and lepirudin
***treatments for gout:***
   including probenecid and sulfinpyrazone
***COPD and asthma treatments:***
   including ipratropium
***treatments for osteoporosis:***
   including raloxifene, pamidronate and risedronate.

Particularly preferred biologically active compounds include lidocaine, diclofenac, ketoralac, prilocaine, halobetasol, hydrocortisol and combinations thereof.

It is to be understood that pharmaceutically acceptable derivatives of biologically active compounds are included within the scope of the present invention.

The term "pharmaceutically acceptable derivatives" includes, but is not limited to, pharmaceutically acceptable salts, esters, salts of such esters, ethers, or any other derivative including prodrugs and metabolites, which upon administration to a subject in need is capable of providing, directly or indirectly, a biologically active compound as otherwise described herein.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describe pharmaceutically acceptable salts in detail in J. Pharmaceutical Sciences, 66:1-19, 1977. Examples of pharmaceutically acceptable nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid, or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hernisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate, and aryl sulfonate.

The term "pharmaceutically acceptable ester" refers to esters which are hydrolysed *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formates, acetates, propionates, butyrates, acrylates and ethylsuccinates.

The term "pharmaceutically acceptable prodrugs" as used herein refers to those prodrugs of the biologically active compounds which are, within the scope of sound medical judgment, suitable for use in contact with the tissues of a subject with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987.

A biologically active compound may be present in an amount of up to about 30%w/w of the total concentration of the carrier composition. The amount of biologically active compound is preferably up to about 10%w/w, more preferably up to about 6%w/w, most preferably within the range of about 0.1%w/w to about 5%w/w.

The carrier composition improves the delivery of a biological active compound by altering the A.D.M.E. properties of the biological active compound. Not wishing to be bound by theory, it is believed that the A.D.M.E. properties of a biological active compound are altered because the combination of a phosphate compound of an electron transfer agent and a relatively high concentration of a polar protic solvent changes the solubility of the biologically active compound in different barriers (skin, mucous membranes, muscle, and so on). This solubility change affects the residence time of the drug in these tissues and delays, or shortens, the time at which the drug will be introduced to the elimination phase. Accordingly, a carrier composition of the present invention can provide the advantage of:
- reduced side effects (i.e. minimalising the unrequired systemic effect);
- restricting the biologically active compound distribution to specific areas (e.g. drug-targeting);
- improving patient compliance (e.g. less amount used, smaller application);
- improving aesthetic feeling (e.g. fast-drying); and
- increasing duration of action and/or shorten onset.

A carrier composition of the present invention may also improve the bioavailability of a biologically active compound in a subject.

Also disclosed herein is a method for treating a subject for a pathological condition which comprises administering an effective amount of a biologically active compound in a carrier composition of the present disclosure. The pathological conditions include those that can be treated by the biologically active compound formulated with the carrier composition.

The term "subject" as used herein refers to any animal having symptoms associated with or caused by a pathological condition, which requires treatment with a biologically active compound. The animal may be a mammal, preferably a human, or may be a non-human primate or non-primates such as used in animal model testing. While it is particularly contemplated that a formulation of the invention is suitable for use in medical treatment of humans, it is also applicable to veterinary treatment, including treatment of companion animals such as dogs and cats, and domestic animals such as horses, ponies, donkeys, mules, llama, alpaca, pigs, cattle and sheep, or zoo animals such as primates, felids, canids, bovids, and ungulates.

Generally, the terms "treating", "treatment" and the like are used herein to mean affecting a subject, tissue or cell to obtain a desired pharmacological and/or physiological effect. The effect may be prophylactic in terms of completely or partially preventing one or more pathological conditions, and/or may be therapeutic in terms of a partial or complete cure of one or more pathological conditions.

### Routes of administration

Routes of administration can broadly be divided into a three categories by effect, namely, "topical" where the desired effect is local, so the substance is applied directly where its action is desired, "enteral" where the desired effect is systemic (non-local) so the substance is given via the digestive tract, and "parenteral" where the desired effect is systemic, so the substance is given by routes other than the digestive tract.

The U.S. Food and Drug Administration recognise 111 distinct routes of administration. The following is a non-limiting list of examples of routes of administration.

Examples of topical routes of administration having a local effect include epicutaneous (onto the skin) and intravitreal (onto the eye).

Examples of enteral routes of administration having a systemic (non-local) effect include any form of administration that involves any part of the gastrointestinal tract, such as oral (into the mouth), intranasal (into the nose), rectal (into the rectum), and vaginal (into the vagina).

Examples of parenteral routes of administration by injection, infusion or diffusion having a systemic effect include intravenous (into a vein), intraarterial (into an artery), intramuscular (into a muscle), intracardiac (into the heart), subcutaneous (under the skin), percutaneous (via needle-puncture into the skin), intradermal (into the skin itself), intrathecal (into the spinal canal), intraperitoneal (infusion or injection into the peritoneum), intravesical infusion (infusion into the urinary bladder), epidural (injection or infusion into the epidural space), transdermal or transcutaneous (diffusion through the intact skin), transmucosal (diffusion through a mucous membrane), insufflation (diffusion through the nose), inhalational (diffusion through the mouth), sublingual (under the tongue), and buccal (absorbed through cheek near gumline).

Formulations according to the present invention can be in any suitable administration form (see, for example, Pharmaceutics and Pharmacy Practice, J. B. Lippincott Company, Philadelphia, Pa., Banker and Chalmers, eds., pages 238-250 (1982)). Examples of suitable administration forms includes, but are not limited to, solutions, liquids, suspensions, gels, poultices, reservoir patches, and creams. The formulations may also be prepared and stored in one form and delivered in another, for example the formulation may be stored in liquid form and delivered in the form of a foam. The formulations and can be prepared by any methods well known in the art of pharmacy such as described in Remington J. P., The Science and Practice of Pharmacy, ed. A. R. Gennaro, 20th edition, Lippincott, Williams and Wilkins Baltimore, Md. (2000). Such methods include the step of bringing into association the biologically active compound with the carrier, and then, if necessary, shaping the formulation into the desired product.

### Preparation of a carrier composition

A carrier composition of the present invention may be prepared by a variety of techniques.

One method of preparing the carrier composition involves combining the components of the carrier composition, in suitable quantities, with stirring, until complete homogenisation is achieved.

In a preferred method, a phosphate compound of an electron transfer agent is combined with a polar protic solvent, and warmed at 40°C until a solution is formed. An aqueous phase, usually of lower volume, is heated to 40°C and then added drop wise to the solution to form the carrier composition. Alternately in some circumstances the phosphate compound of the electron transfer agent combined with the polar protic solvent may be added dropwise to the aqueous phase. The final pH of the carrier composition may be adjusted to improve stability, for example by the addition of sodium hydroxide. The carrier composition is usually a clear to translucent solution.

Depending on the solubility and stability of the biologically active compound, it may be dissolved in either the aqueous or solvent phase.

The carrier composition may optionally further comprise one or more excipients. A person skilled in the art of the invention would appreciate suitable excipients which could be included with a carrier composition or a formulation of the present invention. The choice of other excipients will depend on the characteristics of the biologically active compound and the form of administration used. Examples of other excipients include additional solvents such as water, thickeners or gelling agents, surfactants, buffers, emollients, sweeteners, disintegrators, flavours, colours, fragrances, electrolytes, appearance modifiers, film foaming polymers, propellants and the like. Suitable sweeteners include sucrose, lactose, glucose, aspartame or saccharin. Suitable disintegrators include corn starch, methylcellulose, polyvinylpyrrolidone, xanthan gum, bentonite, alginic acid or agar. Suitable gelling agents include hydroxy propyl cellulose (HPC) and carbopol. Suitable flavours include peppermint oil, oil of wintergreen, cherry, orange or raspberry flavouring. Suitable propellants include butane, carbon dioxide, ethane, hydrochloroflurocarbons, isobutane, nitrogen, nitrous oxide, propane, dimethyl ether, isopentanol, pentane and mixtures thereof such as Propellant A-46 (20% propane and 80% isobutane). The relatively high concentration of organic solvent may avoid the need for a further preservative to be added; however if considered necessary, suitable preservatives that may be added include sodium benzoate, methylparaben, propylparaben, and sodium bisulphite.

The amount of excipient or excipients, if present, is preferably up to about 10%w/w, more preferably up to about 5%w/w, most preferably up to about 3%w/w, and even more preferably either 0.01-3%w/w or 0.1-1%w/w of the total concentration of the carrier composition.

The carrier composition and formulation according to the present invention have been found to be physically stable, have no particle size larger than 300nm. There is also no spontaneous sol-gel transformation.

### FIGURES

The examples will be described with reference to the accompanying figures in which:
Figure 1 shows the transdermal delivery of formulations comprising oxycodone in Franz cells *in vitro*;
Figures 2A and 2B show the relative behaviour of formulations comprising oxycodone in a dose response;
Figure 3 shows comparative skin deposition results of formulations comprising lidocaine;
Figure 4 shows comparative skin deposition results of formulations comprising diclofenac; and
Figure 5 shows comparative skin deposition results of formulations comprising ketorolac.

### EXAMPLES

Various embodiments/aspects of the present invention will now be described with reference to the following non-limiting examples.

### Example 1

Carrier compositions were prepared according to the preferred method described above.

Each of the five carrier compositions comprised 1%w/w of a mixture of mono-(tocopheryl) phosphate and di-(tocopheryl) phosphate in a ratio of 8:2 and water, and the following polar protic solvent concentrations:

| | % polar protic solvent |
|---|---|
| (i) | 50%w/v ethanol |
| (ii) | 60%w/v ethanol |
| (ii)) | 70%w/v ethanol |
| (iv) | 80%w/v ethanol |
| (v) | 90%w/v ethanol |

### Method for thermal cycling

The carrier compositions were refrigerated at about 5°C for approximately 12 hours, and then subjected to a temperature of about 30°C for approximately 12 hours for 3 cycles. Between each change of temperature the carrier compositions were left at room temperature for 3 hours and observed for any turbidity and precipitation.

| | Clarity of solution | Precipitate |
|---|---|---|
| (i) | slightly turbid | none |
| (ii) | clear | none |
| (ii)) | clear | none |
| (iv) | clear | none |
| (v) | clear | none |

### Example 2

This example compares percutaneous oxycodone delivery *in vitro* using Formulation 2A and a formulation which comprises a low polar protic solvent concentration (Formulation 2B). Details of the each formulation are as follows:

| Formulation 2A | |
|---|---|
| Components | Amounts |
| A mixture of mono-(tocopheryl) phosphate and di-(tocopheryl) phosphate in a ratio of 6:4 | 1%w/w |
| Isopropanol | 70%w/w |
| Oxycodone | 5%w/w |
| Molecular mass: 315.40 g/mol | |
| Melting point: 218-220°C | |
| Hydroxypropyl cellulose (HPC) H | 1%w/w |
| QS dH₂O | |

| Formulation 2B | |
|---|---|
| Components | Amounts |
| A mixture of mono-(tocopheryl) phosphate and di-(tocopheryl) phosphate in a ratio of 7:3 | 2%w/w |
| Isopropanol | 10%w/w |
| Ethanol | 10%w/w |
| Oxycodone | 1.5%w/w |
| Carbopol NF10 | 0.25%w/w |
| QS dH₂O | |

| | |
|---|---|
| Formulation 2A had a pH of 6. Formulation 2B had a pH of 6.4. | |

### Method

Formulation 2A was prepared according to the preferred method described above. Formulation 2B was similarly prepared.

Full thickness rat abdominal skin was used in 12ml vertical Franz diffusion cells (PermeGear, PA). Rats were killed by asphyxiation using CO₂ gas and the abdominal area carefully shaved and excised. All underlying fat and connective tissue was removed. Skin was frozen flat between sheets of aluminium foil and stored at -20°C until the morning of experimentation.

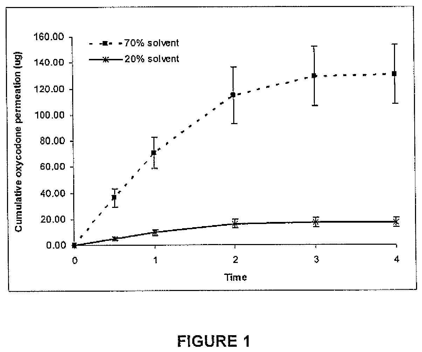

PBS was used in the Franz cells as the receptor solution (12ml) and the Franz cells had a surface area of 1.77cm². During experiments, the cells were maintained at 32°C. Finite dosing (40µl) was used to approximate the conditions to be used *in vivo*. Receiver solutions were sampled regularly over 4 hours to determine the percutaneous oxycodone absorption and analysed using HPLC. The results are presented in Figure 1. The results are the averages of n=8-10 diffusion cells conducted across two separate days. Bars represent SEM.

### Results

It was found that Formulation 2A, which has a polar protic solvent concentration of 70%w/w, could maintain a concentration of oxycodone of 5%w/w. Formulation 2B, which has a polar protic solvent concentration of 20%w/w on the other hand, could only maintain a maximum concentration of oxycodone of 1.5%w/w.

Although both formulations were able to transdermally deliver oxycodone, the results show that, after 4 hours, approximately 130µg of oxycodone was delivered by Formulation 2A compared to approximately 18µg delivered by Formulation 2B.

The drying time for Formulation 2A led to a rapid rate of delivery (i.e. flux), in addition to the increased total amount. The linear flux (J) of percutaneous oxycodone absorption for Formulation 2A was 40µg.h/cm², whereas Formulation 2B had flux of 5.54µg.h/cm².

While both the increased flux and amount of oxycodone delivered may be partially attributed to the increased oxycodone concentration (and subsequent dose), the results show that Formulation 2A also had higher bioavailability. The percentage of oxycodone delivered from the total applied dose was approximately 8% with Formulation 2A compared to only approximately 3% with Formulation 2B.

### Conclusion

Formulation 2A was capable of an increased oxycodone concentration relative to Formulation 2B. Formulation 2A also showed better percutaneous oxycodone absorption, both with respect to the rate and total oxycodone delivered, and superior bioavailability.

### Example 3

This example is a dose response study that compares the performance of Formulation 3A and an aqueous formulation (Formulation 3B) to determine their relative behaviour in a dose response. Details of the each formulation are as follows:

| Formulation 3A | |
|---|---|
| Components | Amounts |
| A mixture of mono-(tocopheryl) phosphate and di-(tocopheryl) phosphate in a ratio of 6:4 | 1%w/w |
| Isopropanol | 70%w/w |
| Oxycodone | 5%w/w |
| Molecular mass: 315.40 g/mol | |
| Melting point: 218-220°C | |
| HPC H | 1%w/w |
| QS dH₂O | |

| Formulation 3B | |
|---|---|
| Components | Amounts |
| A mixture of mono-(tocopheryl) phosphate monosodium salt and di-(tocopheryl) phosphate in a ratio of 2:1 | 10.8%w/w |
| Oxycodone.HCl | 0.7%w/w |
| Molecular mass: 351.83 g/mol | |
| Pemulen | 0.7%w/w |
| QS dH₂O | |

Formulation 3A had a pH of 6. Formulation 3B had a pH of 8.

### Method

Formulation 3A was prepared according to the preferred method described above. Formulation 3B was similarly prepared.

Full thickness rat abdominal skin was used in 12ml vertical Franz diffusion cells (PermeGear, PA). Rats were killed by asphyxiation using CO₂ gas and the abdominal area carefully shaved and excised. All underlying fat and connective tissue was removed. Skin was frozen flat between sheets of aluminium foil and stored at -20°C until the morning of experimentation.

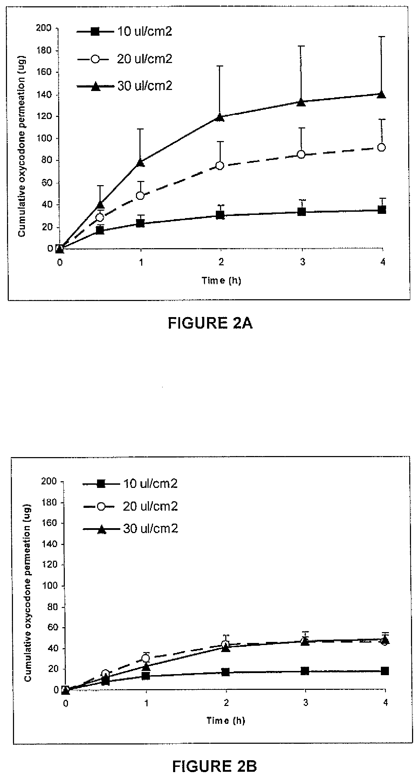

PBS was used in the Franz cells as the receptor solution (12ml) and the Franz cells had a surface area of 1.77cm². During experiments, the cells were maintained at 32°C. Finite dosing (20-60µl) was used to approximate the conditions to be used *in vivo*. Receiver solutions were sampled regularly over 4 hours to determine the percutaneous oxycodone absorption and analysed using HPLC. The results are presented in Figures 2A and 2B.

### Results

Formulation 3A had increased transdermal flux compared to Formulation 3B. The use of a high polar protic solvent concentration (Formulation 3A) allowed equivalent amounts of the preferred form of oxycodone (base; 5%w/w) to be formulated.

With equivalent doses between 10-20µl/cm², Formulation 3A delivered twice as much oxycodone as Formulation 3B, with both formulations exhibiting a good dose response. At a dose of 30µl/cm², no further increase in transdermal delivery is seen with Formulation 3B. The dose response continues for Formulation 3A even at the highest dose of 30µl/cm².

### Conclusion

This dose response study particularly shows that Formulation 3A allows for wider dynamic range in dose responses.

### Example 4

This example compares the stability, skin deposition and other properties of Formulation 4A and a formulation which comprises a low polar protic solvent concentration (Formulation 4B). Details of the each formulation are as follows:

| Formulation 4A | |
|---|---|
| Components | Amounts |
| A mixture of mono-(tocopheryl) phosphate and di-(tocopheryl) phosphate in a ratio of 8:2 | 1%w/w |
| Ethanol | 80%w/w |
| Lidocaine base | 5%w/w |
| Molecular mass: 234.34 | |
| Melting point: 68°C | |
| Hydroxypropyl cellulose(GPHX grade, where G is the Molecular mass grade, PHX means pharmaceutical grade.) | 3%w/w |
| QS dH₂O | |

| Formulation 4B | |
|---|---|
| Components | Amounts |
| A mixture of mono-(tocopheryl) phosphate and di-(tocopheryl) phosphate in a ratio of 2:1 | 1%w/w |
| Ethanol | 30%w/w |
| Lidocaine base | 1%w/w |
| Molecular mass: 234.34 | |
| Melting point: 68°C | |
| Carbopol (CP934) | 1%w/w |
| QS dH₂O | |

### Method

Formulation 4A was prepared according to the preferred method described above. Formulation 4B was similarly prepared.

### Results

The results of a visual stability test showed that, after 3 days of storage, there were no physical changes noted with Formulation 4A whereas a precipitate had formed with Formulation 4B.

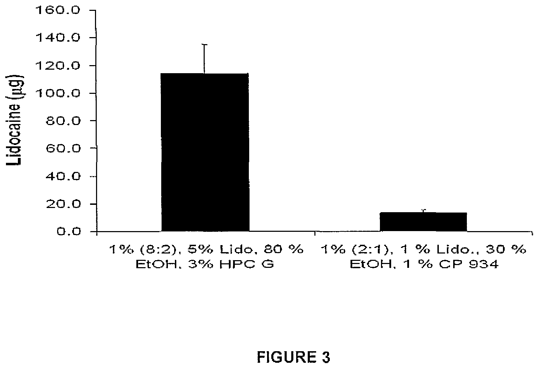

After 4 hours of administration, Formulation 4A had a significant skin deposition (µg) compared to Formulation 4B, as shown in Figure 3.

### Conclusion

The results showed that Formulation 4A was stable and had improved delivery compared to Formulation 4B.

### Example 5

This example compares the stability, skin deposition and other properties of Formulation 5A and a formulation which comprises a low polar protic solvent concentration (Formulation 5B). Details of the each formulation are as follows:

| Formulation 5A | |
|---|---|
| Components | Amounts |
| A mixture of mono-(tocopheryl) phosphate and di-(tocopheryl) phosphate in a ratio of 6:4 | 1%w/w |
| Isopropanol | 70%w/w |
| Diclofenac diethylamine | 2%w/w |
| Hydroxypropyl cellulose (GPHX grade, where G is the Molecular mass grade, PHX means pharmaceutical grade.) | 3%w/w |
| QS dH₂O | |

| Formulation 5B | |
|---|---|
| Components | Amounts |
| A mixture of mono-(tocopheryl) phosphate and di-(tocopheryl) phosphate in a ratio of 6:4 | 1%w/w |
| Isopropanol | 10%w/w |
| Diclofenac diethylamine | 1%w/w |
| Hydroxypropyl cellulose (GPHX grade, where G is the Molecular mass grade, PHX means pharmaceutical grade.) | 0.6%w/w |
| QS dH₂O | |

### Method

Diclofenac Formulation 5A was prepared according to the preferred method described above. Formulation 5B was similarly prepared.

### Results

The results of a visual stability test showed that, after 3 days of storage, there were no physical changes noted with Formulation 5A whereas a precipitate had formed with Formulation 5B.

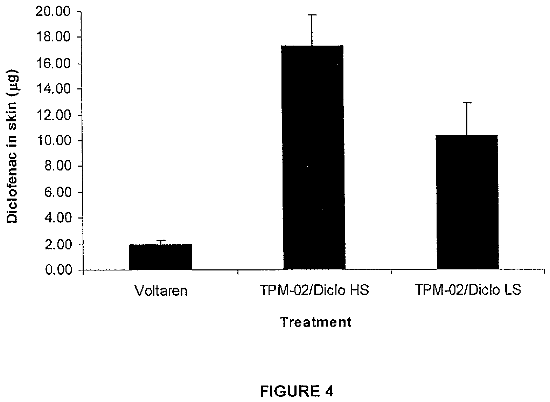

After 4 hours of administration, Formulation 5A had a significant skin deposition (µg) compared to Formulation 5B, as shown in Figure 4. In Figure 4, Formulations 5A and 5B are also compared to the commercially available product comprising diclofenac Voltaren®.

### Conclusion

The results showed that Formulation 5A was stable and had improved delivery compared to Formulation 5B.

### Example 6

This example compares the stability, skin permeation and other properties of Formulation 6A and a formulation which comprises a low polar protic solvent concentration (Formulation 6B). Details of the each formulation are as follows:

| Formulation 6A | |
|---|---|
| Components | Amounts |
| A mixture of mono-(tocopheryl) phosphate and di-(tocopheryl) phosphate in a ratio of 6:4 | 1%w/w |
| Isopropanol | 70%w/w |
| Ketorolac tromethamine | 2%w/w |
| Hydroxypropyl cellulose (GPHX grade, where G is the Molecular mass grade, PHX means pharmaceutical grade) | 3%w/w |
| QS dH₂O | |

| Formulation 6B | |
|---|---|
| Components | Amounts |
| A mixture of mono-(tocopheryl) phosphate and di-(tocopheryl) phosphate in a ratio of 6:4 | 1%w/w |
| Isopropanol | 10%w/w |
| Ketorolac tromethamine | 2%w/w |
| Hydroxypropyl cellulose (GPHX grade, where G is the Molecular mass grade, PHX means pharmaceutical grade) | 0.6%w/w |
| QS dH₂O | |

### Method

Formulation 6A was prepared according to the preferred method described above. Formulation 6B was similarly prepared.

A skin permeation test similar to that conducted for Example 2 was also conducted with the formulations.

### Results

The results of a visual stability test showed that, after 3 days of storage, there were no physical changes noted with Formulation 6A whereas a precipitate had formed with Formulation 6B.

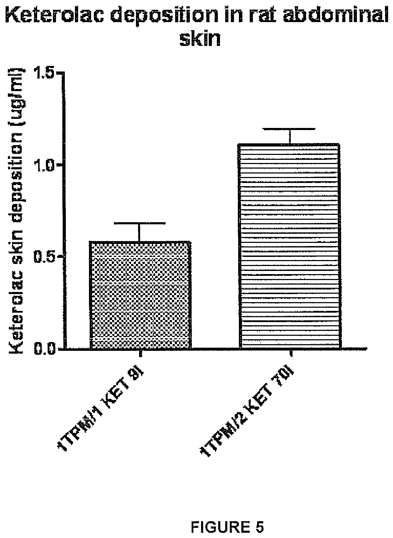

After 4 hours of administration, Formulation 6A had a significant skin deposition (µg) compared to Formulation 6B, as shown in Figure 5.

### Conclusion

The results showed that Formulation 6A was stable and had improved delivery compared to Formulation 6B.

### Example 7

This example compares the stability and other properties of Formulation 7A and a formulation which comprises a low polar protic solvent concentration (Formulation 7B). Details of the each formulation are as follows:

| Formulation 7A | |
|---|---|
| Components | Amounts |
| A mixture of mono-(tocopheryl) phosphate and di-(tocopheryl) phosphate in a ratio of 6:4 | 1%w/w |
| Isopropanol | 70%w/w |
| Felodipine | 1%w/w |
| QS dH₂O | |

| Formulation 7B | |
|---|---|
| Components | Amounts |
| A mixture of mono-(tocopheryl) phosphate and di-(tocopheryl) phosphate in a ratio of 2:1 | 1%w/w |
| Isopropanol | 10%w/w |
| Felodipine | 1%w/w |
| Hydroxypropyl cellulose (GPHX grade, where G is the Molecular mass grade, PHX means pharmaceutical grade.) | 3%w/w |
| QS dH₂O | |

### Method

Formulation 7A was prepared according to the preferred method described above. Formulation 7B was similarly prepared.

### Results

The results of a visual stability test showed that, after 3 days of storage, there were no physical changes noted with Formulation 7A whereas a precipitate had formed with Formulation 7B.

After 4 hours of administration, Formulation 7A had a significant skin deposition (µg) compared to Formulation 7B.

### Conclusion

The results showed that Formulation 7A was stable and had improved delivery compared to Formulation 7B.

## Claims

1. A carrier composition for delivery of a biologically active compound, comprising:
i) a phosphate compound of an electron transfer agent which is a mixture of a mono-(tocopheryl) phosphate and a di-(tocopheryl) phosphate; and
ii) a polar protic solvent, wherein the polar protic solvent is a C₂-C₃ acyclic alcohol; and the polar protic solvent concentration is within the range of 60%w/w to 90%w/w of the total concentration of the carrier composition;
wherein the biologically active compound is lipophilic having a log*P* value within the range of 1 to 5, has a molecular mass of less than 1000 Daltons and has a melting point of less than 400 °C.

2. The carrier composition of claim 1, wherein the polar protic solvent concentration is within the range of 65%w/w to 85%w/w, 70%w/w to 80%w/w, or 60%w/w to 70%w/w, or 80%w/w to 90%w/w.

3. The carrier composition of claim 1, wherein the mixture of a mono-(tocopheryl) phosphate and a di-(tocopheryl) phosphate comprises the sodium or potassium salts of the phosphates.

4. The carrier composition of claim 1, wherein the mixture of a mono-(tocopheryl) phosphate and a di-(tocopheryl) phosphate is in a ratio of at least 2:1, or within the range of 4:1 to 1:4, or within the range of 6:4 to 8:2.

5. The carrier composition of claim 1, wherein the phosphate compound of an electron transfer agent is present in an amount within the range of 0.01%w/w to 20%w/w, or 0.01%w/w to 10%w/w, or 0.01%w/w to 5%w/w, or 0.01%w/w to 2%w/w, or 5%w/w to 10%w/w, or 10%w/w to 15%w/w, or 0.05%w/w, or 0.1 %w/w or 1%w/w of the total concentration of the carrier composition.

6. A formulation comprising a carrier composition of claim 1 and a biologically active compound, wherein the biologically active compound is lipophilic having a log*P* value within the range of 1 to 5 and has a molecular mass of less than about 1000 Daltons and/or a melting point of less than about 400 °C.

7. The formulation of claim 6, wherein the biologically active compound is present in an amount of up to 30%w/w of the total concentration of the carrier composition, or is present in an amount of up to 5%w/w of the total concentration of the carrier composition.

8. The formulation of claim 7, wherein the biologically active compound is selected from the group consisting of lidocaine, diclofenac, ketorolac, prilocaine, halobetasol, hydrocortisor, and combinations thereof.

9. Use of a carrier composition of claim 1 to improve the delivery of a biologically active compound formulated with the carrier composition, or to alter Absorption, Distribution, Metabolism and Excretion properties of a biologically active compound, or to improve the bioavailability of a biologically active compound in a subject.

## Patentansprüche

1. Eine Trägerzusammensetzung zur Abgabe einer biologisch aktiven Verbindung, umfassend:
i) eine Phosphatverbindung eines Elektronenübertragungsmittels, die ein Gemisch aus einem Mono- (Tocopheryl-)phosphat und einem Di- (Tocopheryl-)phosphat ist, und
ii) ein polares protisches Lösungsmittel, wobei das polare protische Lösungsmittel ein acyclischer C₂-C₃-Alkohol ist und die polare protische Lösungsmittelkonzentration im Bereich von 60 % Massenanteil bis 90 % Massenanteil der Gesamtkonzentration der Trägerzusammensetzung liegt;
wobei die biologisch aktive Verbindung lipophil mit einem logP-Wert im Bereich von 1 bis 5 ist, eine Molmasse von weniger als 1000 Dalton aufweist und einen Schmelzpunkt von weniger als 400 °C aufweist.

2. Trägerzusammensetzung nach Anspruch 1, wobei die polare protische Lösungsmittelkonzentration im Bereich von 65 % Massenanteil bis 85 % Massenanteil, 70 % Massenanteil bis 80 % Massenanteil oder 60 % Massenanteil bis 70 % Massenanteil oder 80 % Massenanteil bis 90 % Massenanteil liegt.

3. Trägerzusammensetzung nach Anspruch 1, wobei das Gemisch aus einem Mono-(Tocopheryl-)phosphat und einem Di- (Tocopheryl-)phosphat die Natrium- oder Kaliumsalze der Phosphate umfasst.

4. Trägerzusammensetzung nach Anspruch 1, wobei das Gemisch aus einem Mono-(Tocopheryl-)phosphat und einem Di- (Tocopheryl-)phosphat in einem Verhältnis von mindestens 2:1 oder im Bereich von 4:1 bis 1:4 oder im Bereich von 6:4 bis 8:2 liegt.

5. Trägerzusammensetzung nach Anspruch 1, wobei die Phosphatverbindung eines Elektronenübertragungsmittels in einer Menge im Bereich von 0,01 % Massenanteil bis 20 % Massenanteil oder 0,01 % Massenanteil bis 10 % Massenanteil oder 0,01 % Massenanteil bis 5 % Massenanteil oder 0,01 % Massenanteil bis 2 % Massenanteil oder 5 % Massenanteil bis 10 % Massenanteil oder 10 % Massenanteil bis 15 % Massenanteil oder 0,05 % Massenanteil oder 0,1 % Massenanteil oder 1 % Massenanteil der Gesamtkonzentration der Trägerzusammensetzung vorhanden ist.

6. Formulierung, umfassend eine Trägerzusammensetzung nach Anspruch 1 und eine biologisch aktive Verbindung, wobei die biologisch aktive Verbindung lipophil mit einem logP-Wert im Bereich von 1 bis 5 ist und eine Molekularmasse von weniger als etwa 1000 Dalton und/oder einen Schmelzpunkt von weniger als etwa 400 °C aufweist.

7. Formulierung nach Anspruch 6, wobei die biologisch aktive Verbindung in einer Menge von bis zu 30 % Massenanteil der Gesamtkonzentration der Trägerzusammensetzung vorhanden ist oder in einer Menge von bis zu 5 % Massenanteil der Gesamtkonzentration der Trägerzusammensetzung vorhanden ist.

8. Formulierung nach Anspruch 7, wobei die biologisch aktive Verbindung ausgewählt ist aus der Gruppe bestehend aus Lidocain, Diclofenac, Ketorolac, Prilocain, Halobetasol, Hydrocortisor und Kombinationen davon.

9. Verwendung einer Trägerzusammensetzung nach Anspruch 1 zur Verbesserung der Abgabe einer mit der Trägerzusammensetzung formulierten biologisch aktiven Verbindung oder zur Veränderung der Absorptions-, Verteilungs-, Stoffwechsel- und Ausscheidungseigenschaften einer biologisch aktiven Verbindung oder zur Verbesserung der Bioverfügbarkeit eines biologischen Wirkstoffs bei einem Probanden.

## Revendications

1. Composition porteuse destinée à l'administration d'un composé biologiquement actif, comprenant :
i) un composé de phosphate d'un agent de transfert d'électrons qui est un mélange d'un phosphate de mono-tocophéryle et d'un phosphate de di-tocophéryle ; et
ii) un solvant protique polaire, ledit solvant protique polaire étant un alcool acyclique en C₂ à C₃ ; et la concentration de solvant protique polaire se trouvant dans la plage de 60 % en p/p à 90 % en p/p de la concentration totale de la composition porteuse ;
ledit composé biologiquement actif étant lipophile présentant une valeur log*P* dans la plage de 1 à 5, possédant une masse moléculaire inférieure à 1000 Daltons et possédant un point de fusion inférieur à 400°C.

2. Composition porteuse selon la revendication 1, ladite concentration de solvant protique polaire se trouvant dans la plage de 65 % en p/p à 85 % en p/p, 70 % en p/p à 80 % en p/p, ou 60 % en p/p à 70 % en p/p, ou 80 % en p/p à 90 % en p/p.

3. Composition porteuse selon la revendication 1, ledit mélange d'un phosphate de mono-tocophéryle et d'un phosphate de di-tocophéryle comprenant les sels de sodium ou de potassium des phosphates.

4. Composition porteuse selon la revendication 1, ledit mélange d'un phosphate de mono-tocophéryle et d'un phosphate de di-tocophéryle se trouvant dans un rapport d'au moins 2:1, ou dans la plage de 4:1 à 1:4, ou dans la plage de 6:4 à 8:2.

5. Composition porteuse selon la revendication 1, ledit composé de phosphate d'un agent de transfert d'électrons étant présent en une quantité dans la plage de 0,01 % en p/p à 20 % en p/p, ou 0,01 % en p/p à 10 % en p/p, ou 0,01 % en p/p à 5 % en p/p, ou 0,01 % en p/p à 2 % en p/p, ou 5 % en p/p à 10 % en p/p, ou 10 % en p/p à 15 % en p/p, ou 0,05 % en p/p, ou 0,1 % en p/p ou 1 % en p/p de la concentration totale de la composition porteuse.

6. Formulation comprenant une composition porteuse selon la revendication 1 et un composé biologiquement actif, ledit composé biologiquement actif étant lipophile présentant une valeur log*P* dans la plage de 1 à 5 et possédant une masse moléculaire inférieure à environ 1000 Daltons et/ou un point de fusion inférieur à environ 400°C.

7. Formulation selon la revendication 6, ledit composé biologiquement actif étant présent en une quantité représentant jusqu'à 30 % en p/p de la concentration totale de la composition porteuse, ou étant présent en une quantité représentant jusqu'à 5 % en p/p de la concentration totale de la composition porteuse.

8. Formulation selon la revendication 7, ledit composé biologiquement actif étant choisi dans le groupe constitué par la lidocaïne, le diclofénac, le kétorolac, la prilocaïne, l'halobétasol, l'hydrocortisor, et des combinaisons de ceux-ci.

9. Utilisation d'une composition porteuse selon la revendication 1 pour améliorer l'administration d'un composé biologiquement actif formulé avec la composition porteuse, ou pour modifier les propriétés d'absorption, de distribution, de métabolisme et d'excrétion d'un composé biologiquement actif, ou pour améliorer la biodisponibilité d'un composé biologiquement actif chez un sujet.
